# EUROPEAN PATENT APPLICATION

(11) **EP 4 356 847 A1**
(43) Date of publication of application: **24.04.2024**
(21) Application number: 22201922.6
(22) Date of filing: 17.10.2022
(51) Int. Cl.: A61B 17/04, A61B 17/072, A61B 17/12, A61M 25/00, A61B 17/30

(54) **ENDOVASCULAR CATHETER SYSTEM FOR INVERSION OF THE LAA, PARTICULARLY FOR RESECTION OF THE LAA**

(71) Applicant: BIOTRONIK AG, 8180 Bülach (CH)
(72) Inventor: Nollert, Georg, 82064 Strasslach (DE); Glauser, Thierry, 8121 Benglen (CH)
(74) Representative: Biotronik Corporate Services SE

(57) **Abstract**

The present invention relates to a catheter system (1) for inversion of the left atrial appendage (LAA), comprising an elongated outer sheath (10), an elongated steerable inverting tool (11) slidable in a lumen of the outer sheath (10), the inverting tool (11) configured to cross the atrial septum (S) from the right atrium (RA) towards the left atrium (LA), to engage with the left atrial appendage (LAA), and to invert the left atrial appendage (LAA) upon retracting the inverting tool (11), and an elongated steerable locking tool (13) slidable in a lumen of the outer sheath (10), the locking tool (13) being configured to lock the inverted left atrial appendage (LAA).

## Description

The present invention relates to a catheter system for inversion, and optionally resection, of the left atrial appendage (LAA).

Most patients with atrial fibrillation are anticoagulated for life. Surgically, the left atrial tube can be ligated and resected after thoracotomy. This is a very invasive procedure.

Currently, various occluder systems, which are permanent cardiac implants, are used to fill and occlude the left atrial appendage (LAA) via a transseptal approach. The disadvantages of these devices include continuous thrombi formation in the atrial appendage, which can embolize if the atrial appendage is not completely closed. Furthermore, anticoagulants must be given for a period of time, which is problematic because the indication for this procedure is only for patients with a contraindication to anticoagulants.

Particularly, anticoagulation is always associated with the risk of severe bleeding especially in elderly patients. Particularly, cerebral hemorrhages are feared.

Thoracotomy is a major invasive surgical procedure associated with significant pain and morbidity. Therefore, the surgical intervention is rarely performed for lone atrial fibrillation, but it is performed in combination with other cardiac surgical interventions (e.g., mitral valve repair, VHF ablation, etc.).

Furthermore, the complications of LAA occluders include thromboembolism, device embolism, perforation, etc., so the use is currently limited to a small subgroup of VHF patients who have a contraindication to long-term coagulation due to their increased risk of bleeding.

Based on the above, the problem to be solved by the present invention is to provide a catheter system for inversion of the LAA that allows to reduce implant related complications.

The problem is solved by a catheter system according to claim 1. Preferred embodiments of this aspect of the present invention are stated in the corresponding dependent claims and are described below.

A catheter system for inversion of the left atrial appendage of a human or animal heart being disclosed, comprises:
- an elongated outer sheath,
- a steerable inverting tool slidable in a lumen of the outer sheath, and
- a steerable locking tool slidable in a lumen of the outer sheath.

The inverting tool is configured to engage with the left atrial appendage and to invert the left atrial appendage upon retracting the inverting tool.

The inverting tool comprises a puncturing member. The puncturing member and/or the outer sheath is/are configured to cross the atrial septum (S) from the right atrium (RA) towards the left atrium (LA).

The locking tool is configured to lock the inverted left atrial appendage in place.

According to a preferred embodiment of the present invention, the inverting tool comprises a crossing member comprising a tip at a distal end of the puncturing member configured to cross the atrial septum and further comprises an engaging portion, particularly connected to the tip, the engaging portion being configured to engage with tissue of a far end of the left atrial appendage after having punctured the atrial septum with said tip.

According to a preferred embodiment of the present invention, the engaging portion is a helical portion. Advantageously, such an inverting tool can be removed at the end of the procedure with minimal trauma.

According to a further preferred embodiment of the present invention, the engaging portion comprises at least one barb for inverting the LAA.

According to a preferred embodiment of the present invention, the at least one barb is a retractable barb that is configured to be moved from a retracted position to an anchoring position in which the at least one barb anchors the engaging portion in said tissue of the LAA to allow inversion of the LAA by retracting the inverting tool. Furthermore, according to a preferred embodiment of the present invention, the engaging portion comprises a plurality of barbs. Particularly, the respective barb stays in the inner surface of the LAA such that the inverting tool can be pulled back to invert the LAA into the left atrium.

Once the LAA is secured/locked in the inverted position, the inverting tool can be moved forward and the respective barb retracted before removal through the outer sheath of the catheter system.

Further, according to a preferred embodiment of the present invention, the engaging portion comprises a plurality of flexible protrusions protruding from a base of the engaging portion wherein said protrusions are distributed along a circumferential direction of the base as well as along an axial direction of the base. The plurality of protrusions increases an attractive force between the tissue and the engaging portion (e.g. similar to the hairs of a gecko foot) and thus also allows to invert the LAA once the flexible protrusions are engaged with tissue of the LAA.

Furthermore, according to a preferred embodiment of the present invention, the engaging portion is a forceps that is configured to grab the far end of the LAA. Particularly, the forceps is configured to be locked closed to pull back the LAA into the left atrium. Once the LAA is secured in the inverted position the forceps can be opened to release the tissue and the inverting tool is pulled back into the outer sheath

Further, according to yet another preferred alternative embodiment of the present invention, the engaging portion is a suction cup, wherein the suction cup is configured to be collapsed so that the suction cup can slide through the outer sheath to a far end of the LAA. In a preferred embodiment of the present invention, the suction cup is configured to be opened, particularly by means of a shape memory ribbing or an inflatable ribbing, wherein in said open state, a negative pressure can be applied to the suction cup so that the suction cup can engage with the LAA by sucking at least a portion of the LAA into the suction cup and thereby hold it so that the LAA can be inverted by retracting the suction cup. After the inversion of the LAA, the negative pressure is released, and the collapsed suction cup is pulled back into the outer sheath.

Particularly, the inverted LAA is locked in place using e.g. a suture, staple(s) or an anchor system. To minimize areas where low or no risk of inducing thrombosis is present, the locking is preferably done as close as possible to the original neck of the LAA.

According to a preferred embodiment of the present invention, the locking tool comprises a suture loop to lock the inverted left atrial appendage in place, wherein the suture loop is configured to be opened to allow the LAA to be inverted through the suture loop. Preferably, the suture loop is positioned as close as possible to the neck of the LAA, and the suture loop is closed. If the result is not satisfactory, the loop can be reopened and repositioned. Once the result is satisfactory, the suture loop is locked in place e.g. by a knot or a clip. The suture is cut, and the locking tool is removed.

According to a preferred embodiment of the present invention, the locking tool is a stapler configured to lock the inverted left atrial appendage in place by applying staples to the LAA. Preferably, the locking tool comprises an articulated tip configured to apply said staples. Particularly, the locking tool/stapler is configured to be inserted along the inverting tool, then the articulated tip is turned, e.g. by about 90° and at least one staple is deployed through the LAA wall as close as possible to the neck of the LAA. The stapler is removed through the outer sheath.

Furthermore, according to a preferred embodiment of the present invention, the locking tool is configured to deploy an anchor system comprising at least a first and a second anchor for locking the LAA in place, wherein the looking tool is configured to be passed through opposing walls of the LAA (e.g. in the vicinity of the neck of the LAA), deploy the first (distal) anchor and to be retracted. The locking tool is further configured to deploy the second (proximal) anchor to bring the opposing LAA walls together. This second anchor can be mechanical or a material that cures in-situ. The deployed anchors are released, and the locking tool is retracted.

Further, the catheter system can comprise a resection tool slidable in a lumen of the outer sheath of the catheter system, wherein the resection tool is configured to resect a portion of the inverted and locked left atrial appendage that protrudes into the left atrium.

Yet another aspect of the present invention relates to a method for inverting and locking a left atrial appendage using a catheter system according to the present invention, wherein the left atrial appendage is inverted using the inverting tool and locked using the locking tool.

In the following, embodiments of the present invention, as well as further features and advantages of the present invention shall be described with reference to the Figures, wherein
- Fig. 1: shows an embodiment of a catheter system according to the present invention comprising an inverting tool configured to invert the left atrial appendage by engaging e.g. with a far end of the LAA,
- Fig. 2: shows the inverting tool according to Fig. 1 after inversion of the LAA,
- Fig. 3: shows an embodiment of the catheter system comprising an inverting tool having multiple barbs,
- Fig. 4: shows an embodiment of the catheter system comprising an inverting tool having at least one retractable barb,
- Fig. 5: shows an embodiment of the catheter system comprising an inverting tool having a helical engaging portion for grabbing and inverting the LAA,
- Fig. 6: shows an embodiment of the catheter system comprising an inverting tool in the form of a stapler with articulated tip,
- Fig. 7A-7C: show an embodiment of the catheter system comprising an inverting tool comprising a collapsible/expandable suction cup for engaging with the LAA and inverting the latter,
- Fig. 8: shows an embodiment of the catheter system comprising a locking tool configured to deploy anchors to lock the inverted LAA in place,
- Fig. 9: shows an embodiment of the catheter system comprising a locking tool comprising a suture loop to lock the inverted LAA in place, and
- Fig. 10: shows an embodiment of the catheter system comprising a locking tool in form of a stapler configured to apply staples to lock the inverted LAA in place.

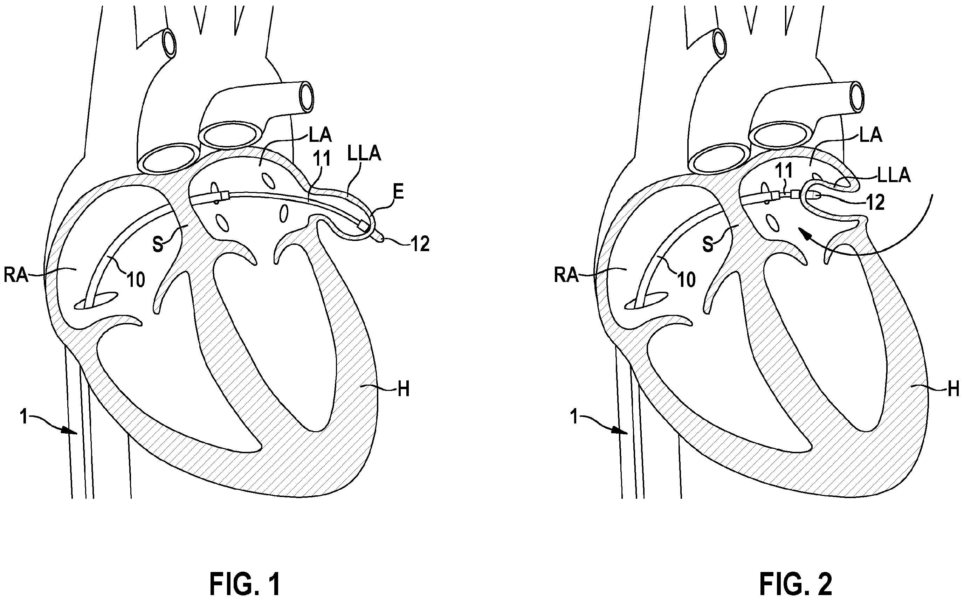

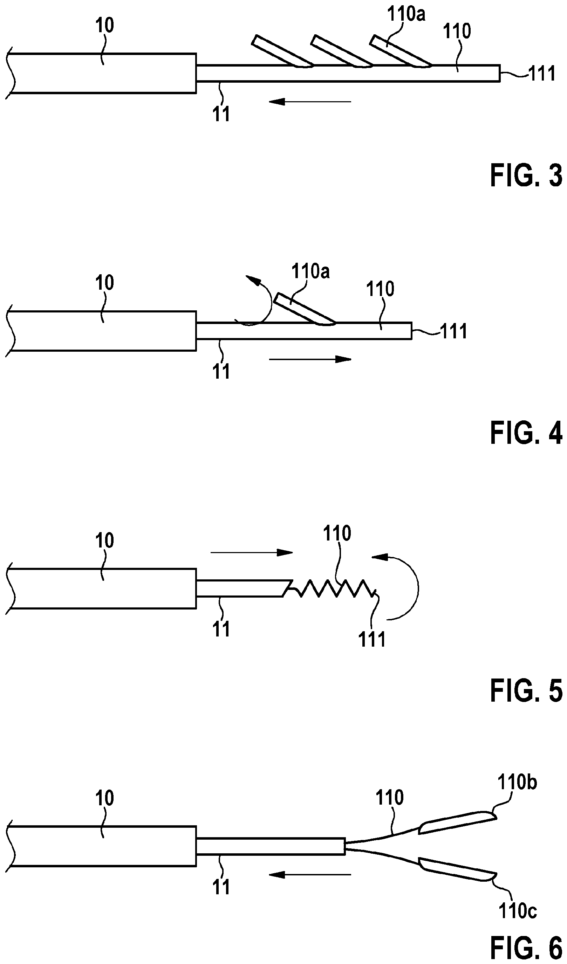

The present invention relates to a catheter system 1 for inversion of the left atrial appendage LAA, e.g. illustrated in Figs. 1 and 2. According thereto, the catheter system 1 comprises an elongated outer sheath 10 and an elongated steerable inverting tool 11 slidable in a lumen of the outer sheath 10, the inverting tool 11 can be configured to puncture the atrial septum S from the right atrium RA towards the left atrium LA, to engage with the left atrial appendage LAA (cf. Fig. 1), and to invert the left atrial appendage LAA upon retracting the inverting tool 11 (cf. Fig. 2). Different embodiments of the inverting tool 11 are shown in Figs. 3 to 7 and will be described below.

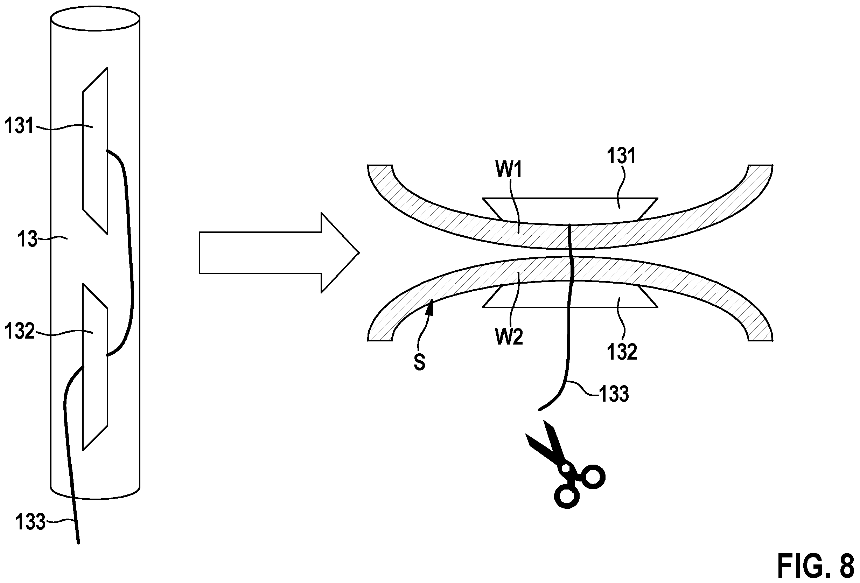

Furthermore, the catheter system comprises an elongated steerable locking tool 13 slidable in a lumen of the outer sheath 10, the locking tool 13 being configured to lock the inverted left atrial appendage LAA. Locking means that two opposing wall sections at a neck of the inverted left atrial appendage LAA are brought close together and are fixed in this position to seal the inverted left atrial appendage LAA. Thereafter, the left atrial appendage LAA can stay in place, particularly in a packaged state, but may also be resected using e.g. a resection tool for resecting and removing the left atrial appendage LAA from the heart. Embodiments of the locking tool 13 are shown in Figs. 8 to 10.

Particularly, the catheter system according to the present invention can be operated as follows:
(a) The outer sheath 10 is passed through the right atrium RA and the inverting tool 11 punches through the atrial septum S into the left atrium LA.
(b) The inverting tool 11 is passed via the catheter into the left atrial appendage LAA.
(c) A far end E of the left atrial appendage LAA is grabbed with the inverting tool and pulled back into the left atrium LA to invert the left atrial appendage LAA.

In case a suture loop 134 is used to lock the inverted left atrial appendage LAA, it is advantageous to invert the left atrial appendage LAA through the opened suture loop in step (c), see also below.

The inverting tool 11 can comprise a puncturing member having a tip 111 at a distal end and may further comprise a helical engaging portion 110 (e.g. a "corkscrew") as shown in Fig. 5 or one or several retractable barbs 110a as shown in Fig. 3 or 4. The engaging portion 110 is passed into the left atrial appendage LAA wall. Particularly, the helical engaging portion 110 can be removed at the end of the procedure with minimal trauma.

In case of an inverting tool 11 with at least one or multiple retractable barbs 110a, the respective barb 110a stays in the inner surface of the left atrial appendage LAA and spikes the tissue (in multiple locations in case several barbs 110a are used) so that the inverting tool 11 can be pulled back along with the left atrial appendage LAA into the left atrium LA to invert the left atrial appendage LAA (as e.g. shown in Fig. 2). Once the left atrial appendage LAA is secured into the inverted position the inverting tool 11 can be moved forward and the barbs 110a retracted before removal through the catheter's outer sheath 10.

Furthermore, as shown in Fig. 6, an inverting tool 11 in form of a forceps can be used, the forceps comprising an engaging portion 110 comprising two members 110b, 110c for grabbing tissue of the left atrial appendage LAA. Particularly, the forceps 11 is moved through the outer sheath 10 until it can grab the far end E of the left atrial appendage LAA. The forceps 11 (e.g. members 110b, 110c) is then locked closed to pull back the left atrial appendage LAA into the left atrium LA. Once the left atrial appendage LAA is secured into the inverted position, the forceps 11 can be opened to release the tissue and the forceps 11 is pulled back into the outer sheath 10 of the catheter.

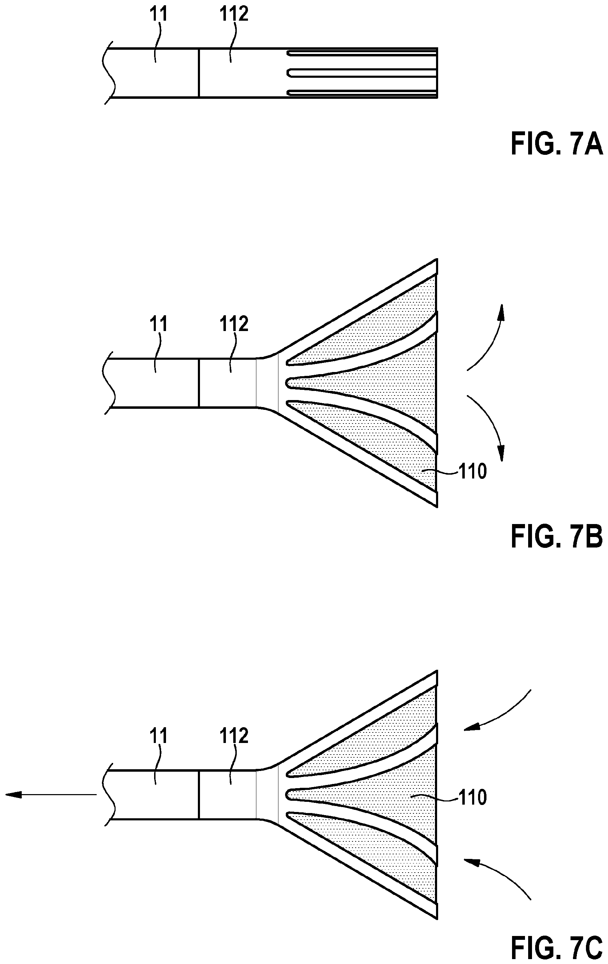

Furthermore, as illustrated in Fig. 7, the inverting tool 11 can comprise a collapsed suction cup 110 as engaging portion that is moved through the outer sheath 10 of the catheter to the far end E of the left atrial appendage LAA. The suction cup 110 may be opened either by its shape memory ribbing 112 or an inflatable ribbing 112 (other mechanisms are also conceivable). The suction cup 110 is positioned, and vacuum is applied through it in order to grab the left atrial appendage LAA tissue. After the procedure is complete the vacuum is released, and the collapsed suction cup 110 is pulled back into the outer sheath 10 of the catheter.

The inverted left atrial appendage LAA is locked in place using a locking tool 13 that may employ a suture, staple or anchor system as indicated in Figs. 8 to 10. To minimize areas where low or even no risks of inducing thrombosis are present, the fixing is preferably done as close as possible to the original neck of the left atrial appendage LAA.

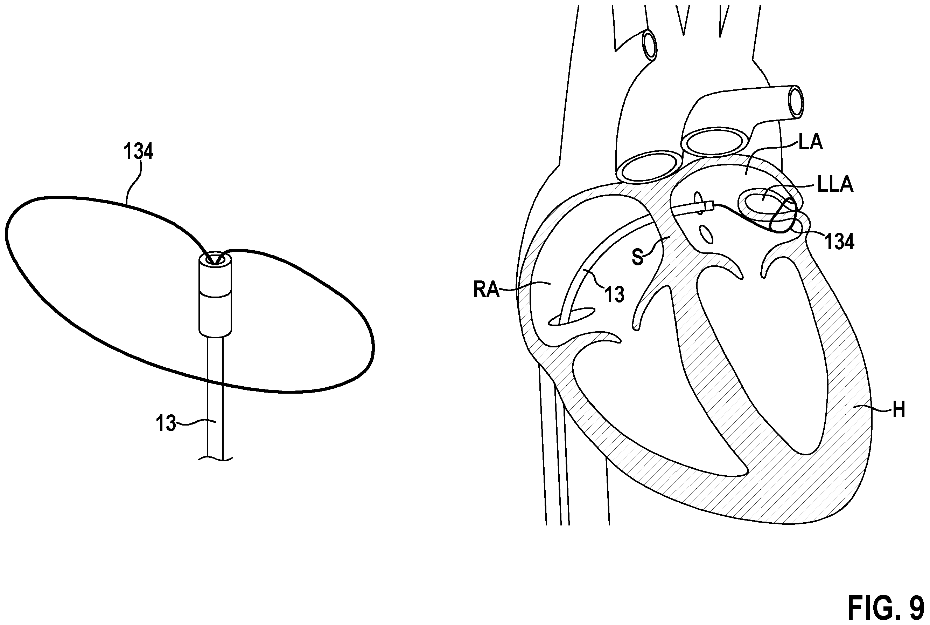

According to the embodiment shown in Fig. 9, the locking tool13 comprises a suture shaped like a lasso (e.g. a suture loop) 134 that is brought first into the left atrial appendage LAA prior to the inversion of the left atrial appendage LAA. The suture loop 134 is then opened, and the left atrial appendage LAA is inverted through this loop 134. The loop 134 is positioned as close as possible to the neck of the left atrial appendage LAA, and the loop 134 is closed. If the result is not satisfactory, the loop 134 can be reopened and repositioned. Once the result is satisfactory the loop 134 is locked in place by a knot or a clip or another means. The suture can be cut, and the locking tool 13 is removed.

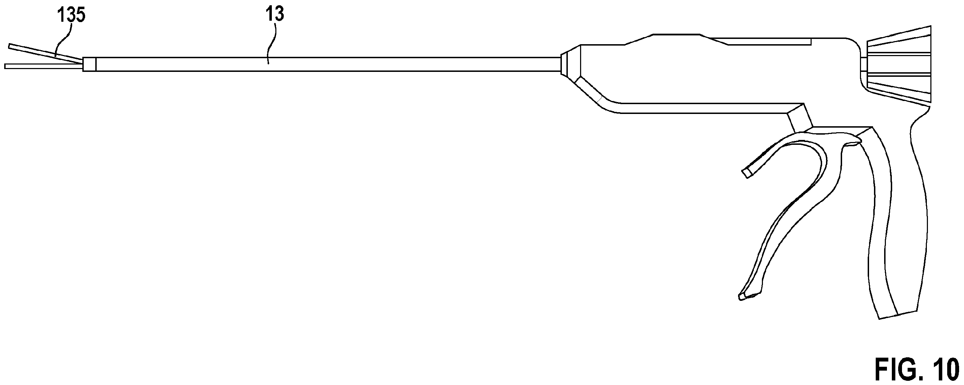

Furthermore, according to Fig. 10 a locking tool 13 in form of a stapler 13 can be inserted along the inverting tool 11 to lock the left atrial appendage LAA in place. The stapler 13 preferably comprises an articulated tip 135 for applying staples to the inverted left atrial appendage LAA. Particularly, the tip 135 is turned 90° and several staples are deployed through the left atrial appendage LAA wall as close as possible to the neck of the left atrial appendage LAA. The stapler 13 is then removed through the outer sheath 10 of the catheter.

According to yet another alternative embodiment of a locking tool 13 shown in Fig. 8, an anchor device 131, 132, 133 is passed into the left atrium through the locking tool 13. It is positioned close to the neck of the inverted left atrial appendage LAA. The locking tool 13 is passed through opposing walls W1, W2 of the left atrial appendage LAA. The first distal anchor 131 is deployed, and the locking tool 13 is pulled back. The second proximal anchor 132 is deployed and positioned to bring the opposing walls W1, W2 of the left atrial appendage LAA together. This proximal anchor 132 can be mechanical or a material that cues in-situ. The deployed anchor 132 is released, and the locking tool 13 is pulled back. A second anchor system can be deployed if the released ones are sub optimal.

Particularly, the alternative looking tools 13 described above can removed once the results are deemed acceptable.

The advantage of the solution according to the present invention is that no implant remains in the left atrium LA, so that the patient does not have to be anticoagulated as in surgical resection. Furthermore, thrombi and emboli from the left atrial appendage cannot occur during the course of the procedure because it has been locked in place, particularly completely resected.

## Claims

1. A catheter system (1) for inversion of the left atrial appendage (LAA), comprising:
- an elongated outer sheath (10),
- a steerable inverting tool (11) comprising a puncturing member, the steerable inverting tool being slidable in a lumen of the outer sheath (10), and the inverting tool (11) is configured to engage with the left atrial appendage (LAA), and to invert the left atrial appendage (LAA) upon retracting the inverting tool (11), and
- a steerable locking tool (13) slidable in a lumen of the outer sheath (10), the locking tool (13) being configured to lock the inverted left atrial appendage (LAA), wherein the puncturing member and/or the outer sheath is/are configured to cross the atrial septum (S) from the right atrium (RA) towards the left atrium (LA).

2. The catheter system according to claim 1, wherein the puncturing member comprises a tip (111) at a distal end configured to cross the atrial septum (S) and further comprises an engaging portion (110) connected to the tip (111), the engaging portion (110) being configured to engage with tissue of a far end (E) of the left atrial appendage (LAA) after having punctured said atrial septum (S) with said tip (111).

3. The catheter system according to claim 2, wherein the engaging portion (110) is a helical portion (110).

4. The catheter system according to claim 2, wherein the engaging portion (110) comprises at least one barb (110a).

5. The catheter system according to claim 4, wherein the at least one barb (110a) is a retractable barb (110a) that is configured to be moved between a retracted position and an anchoring position in which the at least one barb (110a) anchors the engaging portion (110) in said tissue.

6. The catheter system according to claim 4 or 5, wherein the engaging portion (110) comprises a plurality of barbs (110a).

7. The catheter system according to claim 2, wherein the engaging portion comprises a plurality of flexible protrusions for engaging with the left atrial appendage (LAA), said protrusions protruding from a base of the engaging portion wherein said protrusions are distributed along a circumferential direction of the base as well as along an axial direction of the base.

8. The catheter system according to claim 2, wherein the engaging portion (110) is a forceps (110) that is configured to grab a far end (E) of the left atrial appendage (LAA) to invert the left atrial appendage (LAA).

9. The catheter system according to claim 2, wherein the engaging portion (110) is a suction cup (110), wherein the suction cup (110) is configured to be collapsed so that the suction cup (110) can slide through the outer sheath (10) towards a far end (E) of the left atrial appendage (LAA), and wherein the suction cup (110) is configured to be opened, wherein in said open state, a negative pressure is applicable to the suction cup (110) so that the suction cup (110) engages with the left atrial appendage (LAA) by sucking at least a portion of the left atrial appendage (LAA) into the suction cup (110) and thereby hold it so that the left atrial appendage (LAA) can be inverted by retracting the suction cup (110).

10. The catheter system according to one of the preceding claims, wherein the locking tool (13) comprises a suture loop (134) to lock the inverted left atrial appendage (LAA), wherein the suture loop (134) is configured to be opened to allow the left atrial appendage (LAA) to be inverted through the suture loop (134), and wherein the suture loop (134) is configured to be tightened to lock the inverted left atrial appendage (LAA).

11. The catheter system according to one of the claims 1 to 9, wherein the locking tool (13) is a stapler configured to lock the inverted left atrial appendage (LAA) by applying staples to the left atrial appendage (LAA), wherein the locking tool (13) comprises an articulated tip (135) configured to apply said staples.

12. The catheter system according to one of the claims 1 to 9, wherein the locking tool (13) is configured to deploy an anchor system comprising a first and a second anchor (131, 132) configured to bring two opposing wall sections (W1, W2) of the inverted left atrial appendage (LAA) together to lock the inverted left atrial appendage (LAA).
